(19) Europäisches Patentamt
European Patent Office
Office européen des brevets

(11) Numéro de publication: **0 032 713**
**B1**

(12) # FASCICULE DE BREVET EUROPEEN

(45) Date de publication du fascicule du brevet: **18.07.84**

(51) Int. Cl.³: **C 07 F 9/54**, C 07 C 13/32

(21) Numéro de dépôt: **81100199.9**

(22) Date de dépôt: **13.01.81**

(54) **Nouveaux sels de phosphonium, procédé pour leur préparation et leur utilisation à titre de produits de départ pour la préparation de composés bicycliques insaturés.**

(30) Priorité: **16.01.80 CH 332/80**

(43) Date de publication de la demande:
**29.07.81 Bulletin 81/30**

(45) Mention de la délivrance du brevet:
**18.07.84 Bulletin 84/29**

(84) Etats contractants désignés:
**CH DE FR GB IT LI NL SE**

(56) Documents cités:
**CH - A - 519 454**
**DE - A - 2 916 418**

**JOURNAL OF THE AMERICAN CHEMICAL SOCIETY, vol. 97, no. 18, 3 septembre 1975, pages 5497-5507 H.E. ZIMMERMAN: "Conformation and Di-P-methane Reactivity. Mechanistic and Exploratory Organic Photochemistry"**

**JOURNAL OF THE AMERICAN CHEMICAL SOCIETY, vol. 79, no. 20, 22 octobre 1957, pages 5558-64 K. BIEMANN et al.: "The Structure and Synthesis of Muscopyridine"**

(73) Titulaire: **FIRMENICH SA**
**Case postale 239**
**CH-1211 Genève 9 (CH)**

(72) Inventeur: **Uijttewaal, Arnoldus**
**63, rue Liotard**
**CH-1202 Genève (CH)**
Inventeur: **Snowden, Roger**
**5, chemin des Palettes**
**CH-1212 Grand-Lancy/Ge (CH)**

(74) Mandataire: **Schmied-Kowarzik, Volker, Dr. et al,**
**Siegfriedstrasse 8**
**D-8000 München 40 (DE)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

EP 0 032 713 B1

## Description

L'invention se rapport à des sels de phosphonium nouveaux, utiles à titre de produits de départ pour la préparation de composés bicycliques insaturés. Elle a également pour objet un procédé pour leur préparation.

L'invention a plus précisément pour objet des composés de formule

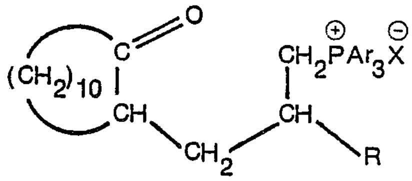

(I)

dans laquelle le symbole R représente un atome d'hydrogène ou un radical méthyle, le symbole Ar sert à désigner un radical aryle et X un atome d'halogène ou un radical $BF_4$ ou $ClO_4$.

Elle a également pour objet un procédé pour la préparation dudit composé de formula (I), caractérisé en ce que

A) on fait réagir un composé de formule

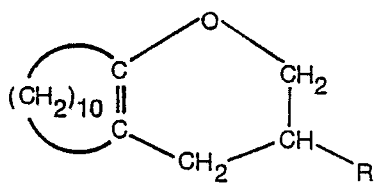

(II)

dans laquelle le symbole R est défini comme indiqué ci-dessus avec un dérivé du phosphore de formule

$$HPAr_3X \qquad (III)$$

dans laquelle le symbole Ar sert à désigner un radical aryle et X est défini comme indiqué ci-dessus; ou

B) on fait réagir un composé de formule

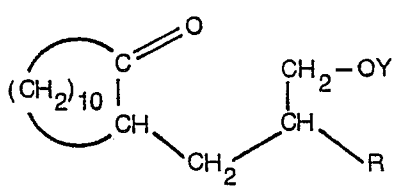

(IV)

dans laquelle le symbole R est défini comme indiqué précédemment et Y représente un radical tert-butyle, tétrahydropyranyle ou trialkyl-silyle, avec le dérivé du phosphore de formule (III) défini ci-dessus; ou

C) on fait réagir un composé de formule

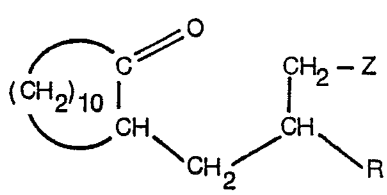

(V)

dans laquelle le symbole R est défini comme indiqué précédemment et Z représente un atome d'halogène, avec une triaryl-phosphine.

Elle a en outre pour objet l'utilisation dudit composé de formule (I) à titre de produit de départ pour la préparation d'un composé bicyclique insaturé de formule

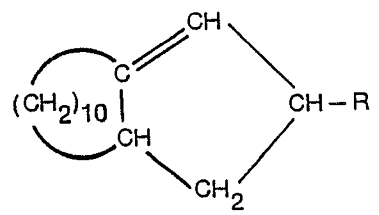

(VI)

dans laquelle le symbole R représente un atome d'hydrogène ou un radical méthyle, caractérisée en ce qu'on traite ledit composé de formule (I) au moyen d'une base.

EXALTONE® et muscone sont toutes deux des cétones macrocycliques fort appréciées dans l'art de la parfumerie pour leur odeur musquée. Connues depuis fort longtemps déjà, elles ont suscité de tout temps l'intérêt des chercheurs, au vu du grand nombre de synthèses publiées dans la littérature scientifique [voir par exemple J. Chem. Soc. *1964*, 4154; Tetrahedron *20*, 2601 (1964); Helv. Chem. Acta *50*, 705 (1967); Helv. Chim. Acta *50*, 708 (1967)].

Toutes ces méthodes cependant ne s'appliquent qu'avec difficulté à l'échelle industrielle, soit à cause de leur complexité, soit à cause du faible rendement de certaines étapes clés.

L'une des méthodes synthétique connues à ce jour [voir Helv. Chim. Acta *50*, 705 (1967)] fait appel au composé de formule

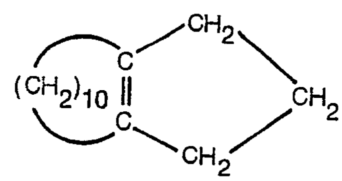

à titre de produit intermédiaire dans la synthèse d'EXALTONE® et à son homologue méthylé, de formule

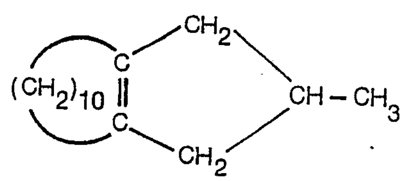

pour la préparation de muscone. Ces composés s'obtiennent à partir de cyclododécanone, après toute une série d'étapes réactionnelles faisant notamment intervenir condensation, cyclisation, hydrogénation et déshydrogénation. Il s'en suit un rendement global relativement bas et de ce fait peu d'intérêt du point de vue industriel.

Les deux composés ci-dessus peuvent être en fait obtenus de façon beaucoup plus rationnelle, par conséquent plus intéressante pour l'industrie, à partir des composés bicycliques de formule (VI) définis précédemment, après traitement de ces derniers au moyen d'un agent isomérisant tel un acide fort minéral ou organique par exemple.

Selon le procédé de l'invention, variante A, le composé oxygéné de formule (II) est premièrement soumis au traitement d'un dérivé du phosphore de formule (III). A titre de composé de formule (III), on utilise de préférence un dérivé de la triphényl-phosphine (Ar = $C_6H_5$ dans la formule III), plus particulièrement le bromure ou le tétrafluoroborate d'hydrogéno-triphényl-phosphonium. Ces composés s'obtiennent selon les méthodes usuelles, à partir de triphényl-phosphine et de l'acide correspondant, par exemple selon Helv. Chim. Acta *45*, 541 (1962) ou Synthesis *1977*, 628.

Ladite réaction peut être effectuée en présence ou non d'un solvant organique inerte, l'avantage de ce dernier étant de permettre un abaissement de la température, par conséquent des conditions réactionnelles plus douces, favorables à l'obtention d'un bon rendement en produit désiré. A titre de solvant, on peut avantageusement utiliser un hydrocarbure ou mélange d'hydrocarbures aromatiques, le toluène ou le xylène par exemple, ou encore le diméthylformamide ou le dioxanne.

Lorsque ladite réaction s'effectue en présence d'un solvant organique inerte tel que ceux définis ci-dessus, on opère à une température supérieure on égale à 110°C environ. Celle-ci peut coïncider, notamment, avec l'ébullition du solvant ou mélange de solvants choisi.

Les composés oxygénés bicycliques de formule (II) utilisés à titre de produit de départ selon le procédé de l'invention sont des composés connus, fabriqués industriellement. Ils peuvent être également préparés selon les prescriptions de la littérature, par exemple selon la méthode publiée dans DE—OS 2.026.056.

Selon une autre mise en oeuvre du procédé de l'invention, variante B, on fait réagir le dérivé du phosphore de formule (III) défini précédemment avec un composé de formule

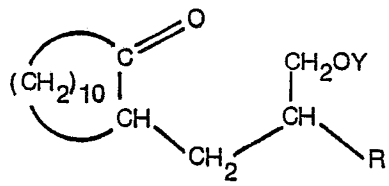

(IV)

(R = H ou $CH_3$)

dans laquelle Y représente un radical tert-butyle, tétrahydropyranyle ou trialkyl-silyle, dans ce dernier cas de préférence triméthyl-silyle. Ledit traitement s'effectue comme indiqué plus haut à propos du composé oxygéné bicyclique de formule (II), de préférence en présence d'un solvant organique inerte tel un hydrocarbure aromatique, le toluène ou le xylène par exemple, à une température supérieure ou égale à 110°C environ.

Selon une autre mise en oeuvre du procédé de l'invention, variante C, on fait réagir une triarylphosphine avec le composé de formule

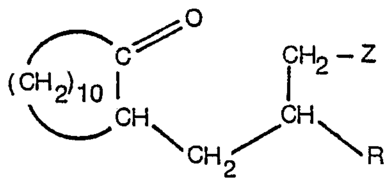

(V)

dans laquelle le symbole Z représente un atome d'halogène, de préférence Br. On utilise de préférence la triphénylphosphine en présence d'un solvant organique inerte tel un éther, un hydrocarbure ou tout autre solvant usuel d'une réaction dite de Wittig. On isole ainsi un sel de phosphonium de formule (I) dans laquelle le symbole X représente un atome d'halogène et Ar représente le radical phényle.

Les composés de formule (IV) et (V) mentionnés ci-dessus peuvent être aisément obtenus à partir du composé hydroxylé correspondant (préparé par exemple selon DE—OS 2.026.056) selon une suite réactionnelle conventionnelle ou, également, à partir de cyclododécanone, après réaction de celle-ci avec le dérivé allylique correspondant, en présence de générateurs de radicaux libres (voir par exemple Synthesis *1976*, 315 et références citées en note). Le schéma réactionnel ci-après illustre les voies réactionnelles que l'on peut utiliser à de telles fins. Dans les formules indiquées, les symboles R, Y et Z utilisés sont définis comme précédemment:

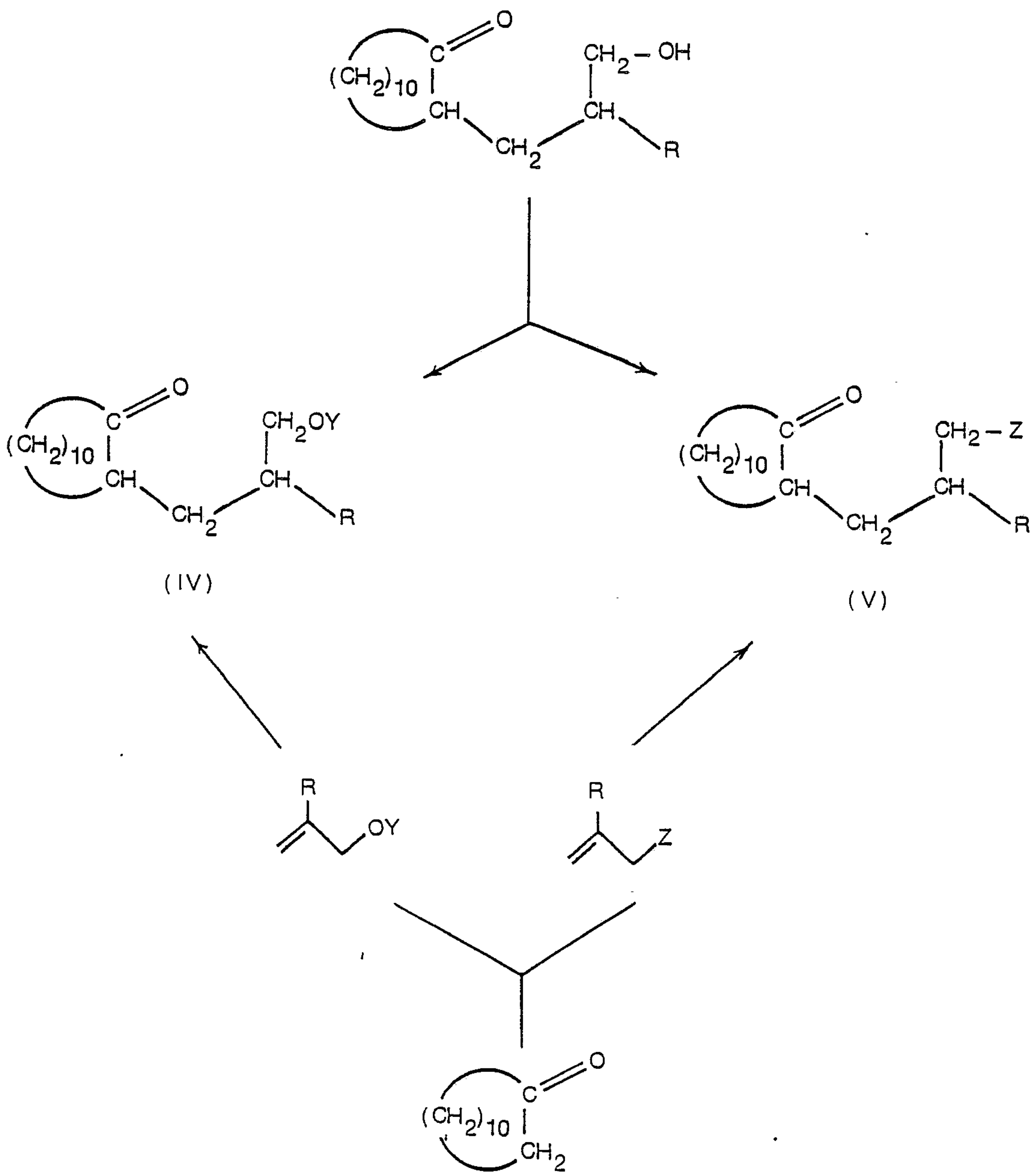

(IV) (V)

Le détail de certaines des préparations mentionnées ci-dessus figure dans les exemples illustrant l'invention.

A titre d'exemples de composés de formule (I) obtenus selon le procédé de l'invention, on peut citer entre autres les composés de formules

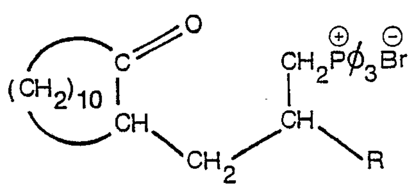

et

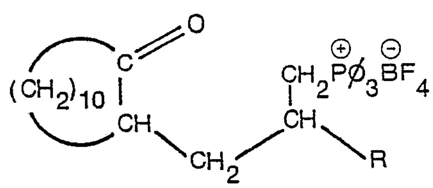

(Ia) (Ib)

R = H ou $CH_3$  $\phi = C_6H_5$

Les composés ci-dessus s'obtiennent sous forme cristalline ou semi-cristalline: ils sont suffisamment stables pour être isolés, analysés et conservés dans les conditions usuelles.

Selon l'invention, les composés de formule (I) peuvent être avantageusement utilisés à titre de produits de départ pour la préparation des composés bicycliques insaturés de formule (VI). Ladite utilisation consiste en un traitement un moyen d'une base.

Ce traitement peut être effectué dans les conditions usuelles d'une réaction de Wittig: on peut notamment recourir à l'emploi d'un hydrure ou d'un alcoolate de métal alcalin, l'hydrure ou le méthylate de sodium, le tert-amylate ou tert-butylate de sodium ou potassium par exemple. On peut également utiliser un alkyl-lithium, le butyl-lithium par exemple ou encore l'amidure de sodium dans l'ammoniac liquide, voire l'hydroxyde de potassium.

Ledit traitement s'effectue en outre en présence d'un solvant organique inerte, le plus généralement sous atmosphère d'azote ou d'argon. A titre de solvant inerte, on peut avantageusement utiliser un hydrocarbure ou mélange d'hydrocarbures aromatiques, un éther ou un amide par exemple. On utilise de préférence le toluène ou le xylène. On opère le plus généralement à température supérieure égale ou à 80°C environ, plus précisément à une température comprise entre 80°C et l'ébullition du solvant ou mélange de solvants choisi. Le composé de formule (VI) ainsi préparé est ensuite isolé du melange de réaction et purifié selon les techniques usuelles, la chromatographie en phase gazeuse ou la distillation fractionnée par exemple.

L'invention sera illustrée de façon plus détaillée à l'aide des exemples ci-après (températures en degrés centigrades).

Example 1
Préparation du composé de formule

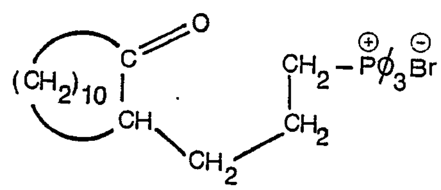

*Variante A*

22,2 g (0,1 mole) de 13-oxa-bicyclo[10.4.0]hexadéc-1(12)ène et 34,5 g (0,1 mole) de bromure d'hydrogèno-triphénylphosphonium — préparé selon Helv. Chim. Acta *45*, 541 (1962) — dans 100 ml de xylène ont été chauffés durant 24 h à reflux. Après refroidissement, décantation du précipité formé, lavages successifs à l'éther, filtration et évaporation, on a recueilli 56,1 g (rendement environ 100%) du composé désiré sous forme cristalline (cristaux blancs), F. 51—85°.

IR: 1710, 1440, 1110 $cm^{-1}$
RMN: 1,2 (14H, m); 1,6 (8H, m); 2,5 (3H, m); 3,8 (2H, m); 7,7 (15H, m) $\delta$ ppm

*Variante B*

14,8 g (0,05 mole) de 2-(3-tert-butoxy-prop-1-yl)cyclododécanone et 17,5 g (0.05 mole) de bromure d'hydrogéno-triphényl-phosphonium dans 100 ml de xylène ont été chauffés durant 70 h à 120°. Aprés refroidissement à température ambiante, évaporation, traitements successifs du résidu au moyen de $CH_2Cl_2$ et d'éther de pétrole (30—50) et finalement évapoaration sous pression réduite, on a recueilli 23,5 g (rendement 83%) du produit désiré.

La 2-(3-tert-butoxy-prop-1-yl)cyclododécanone utilisée ci-dessus comme produit de départ a été obtenue comme suit: 2,8 g (0,025 mole) de 3-tert-butoxy-prop-1-ène et 1,5 g (0.01 mole) de di-tert-butyl-peroxyde ont été ajoutés en 1 h environ à 18,2 g (0,1 mole) de cyclododécanone, maintenus à 140°. Après poursuite du chauffage durant 1 h et finalement distillation fractionnée, on a recueilli le produit désiré avec un rendement de 80%.

Eb. 122°/13,3 Pascals
$n_D^{20}$: 1,4749
RMN: 1,2 (9H, s); 1,3 (14H, m); 1,5 (8H, m); 2,5 (3H, m); 3,3 (2H, m) $\delta$ ppm

*Variante B*

6,24 g (0,02 mole) de 2-(3-triméthyl-silyloxy-prop-1-yl)cyclododécanone dans 50 ml de xylène ont été chauffés durant 50 h à reflux, en présence de 6,8 g (0,02 mole) de bromure d'hydrogéno-triphényl-phosphonium. Après refroidissement à température ambiante, décantation et évaporation sous pression réduite, on a isolé 10,8 g (rendement 96%) du produit désiré, F. 50 à 70° environ.

La 2-(3-triméthyl-silyloxy-prop-1-yl)cyclododécanone utilisée ci-dessus comme produit de départ a été obtenue comme suit: un mélange de 32,5 g (0,25 mole) de 3-triméthyl-silyloxy-prop-1-ène et

14,6 g (0,1 mole) de di-tert-butyl-peroxyde a été ajouté en 6 h à 182 g (1 mole) de cyclododécanone maintenus à 140°. Après distillation fractionnée, on a recueilli le produit désiré avec un rendement de 75% environ.

Eb. 110—125°/6,65 Pascals
$n_D^{20}$: 1,4713
IR: 2899, 1709, 1471, 1414, 1247 $cm^{-1}$
RMN: 0,15 (9H, s); 1,1—1,9 (21H, m); 2,2—2,8 (4H, m); 3,4—3,7 (2H, m) $\delta$ ppm.

*Variante C*

30,3 g (0,01 mole) de 2-(3-bromo-prop-1-yl)-cyclododécanone dans 100 ml de toluène ont été chauffés à reflux durant 24 h, en présence de 26,2 g (0,01 mole) de triphénylphosphine. Après les traitements d'extraction et purification décrits précédemment (variante A), on a isolé 83,3 g du produit désiré (rendement 96%).

La 2-(3-bromo-prop-1-yl)-cyclododécanone utilisée ci-dessus comme produit de départ a été obtenue comme suit:

a) à une solution de 206,7 g (0,86 mole) de 2-(3-hydroxy-prop-1-yl)-cyclododécanone dans 280 ml de pyridine maintenue à 0°, on a ajouté, sous bonne agitation, 171,5 g (0,90 mole) de chlorure de p-toluène-sulfonyle. Après poursuite de l'agitation durant 3 h à 0—10° puis entreposage durant 24 h à 4°, le mélange de réaction a été versé sur glace (1400 g) et acidifié au moyen de 600 ml de HCl à 36% dans $H_2O$. Après extraction à l'éther, séchage sur $CaCl_2$ et évaporation, on a recueilli 273 g (rendement 81%) de 2-(3-p-toluène-sulfonyl-oxy-prop-1-yl)-cyclodocécanone.

IR: 1710, 1605, 1355, 1185, 1175 $cm^{-1}$
RMN: 1,3 (14H, m); 1,5 (8H, m); 2,4 (3H, m); 2,4 (3H, s); 4,0 (2H, t, J=6 Hz); 7,3 (2H, d, J=9 Hz); 7,8 (2H, d, J=9 Hz) $\delta$ ppm

b) 297,3 g (0.75 mole) du produit obtenu ci-dessus dans 1000 ml d'acétone ont été chauffés à reflux durant 24 h, en présence de 90 g (1,0 mole) de bromure de lithium. Après refroidissement, filtration et évaporation de l'excès d'acétone, le mélange de réaction a été additionné de 600 ml de $H_2O$. Après extraction à l'éther, lavage avec une solution aqueuse saturée de $Na_2S_2O_3$, séchage sur $Na_2SO_4$ et évaporation, on a obtenu 213, 1 g (rendement 93%) du produit désiré.

IR: 1710, 1480, 1445, 1245, 725 $cm^{-1}$
RMN: 1,3 (16H, m); 2,7 (6H, m); 2,5 (3H, m); 3,4 (2H, t, J=6 Hz) $\delta$ ppm.

Exemple 2
Préparation du composé de formule

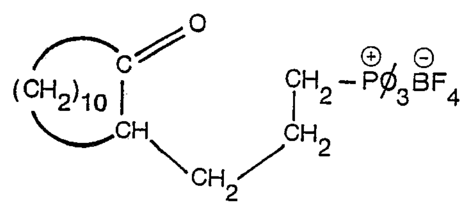

$\phi = C_6H_5$

22,2 g (0,1 mole) de 13-oxa-bicyclo[10.4.0]hexa-déc-1(12)-ène et 35 g (0,1 mole) de tétrafluoroborate d'hydrogéno-triphénylphosphonium — préparé selon Synthesis *1977*, 628 — ont été chauffés durant 1 jour à 170°. Après refroidissement, le mélange de réaction a été repris dans du $CH_2Cl_2$ et le précipité formé lavé à l'éther. Après évaporation sous pression réduite, on a recueilli 52,7 g (rendement 92%) du composé désiré sous forme cristalline (cristaux blancs), F. 57—85°.

IR: 1705, 1440, 1110, 1070 $cm^{-1}$
RMN: 1,2 (14H, m); 1,6 (8H, m); 2,5 (3H, m); 3,2 (2H, m); 7,7 (15H, m) $\delta$ ppm.

Exemple 3
Préparation du composé de formule

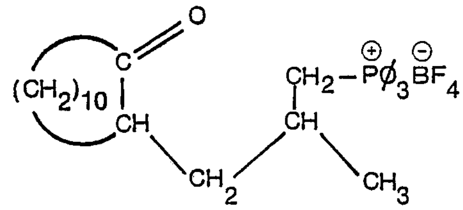

$\phi = C_6H_5$

13,9 g (0,059 mole) de 15-méthyl-13-oxa-bicyclo[10.4.0]hexadéc-1(12)-ène at 20,6 g (0,059 mole) de tétrafluoroborate d'hydrogéno-triphényl-phosphonium ont été chauffés durant 24 h à 170°. Après refroidissement, on a obtenu 34,5 g (rendement 100%) du composé désiré sous forme cristalline, F. 63—69°.

IR: 1710, 1440, 1080 $cm^{-1}$
RMN: 0,9 (3H, d, J=6 Hz); 1,2 (14H, m); 1,6 (7H, m); 2,3 (2H, m); 2,7 (1H, m); 3,2 (2H, dxd, $J_1$=13 Hz, $J_2$=6 Hz); 7,7 (15H, m) $\delta$ ppm.

Le sel de phosphonium ci-dessus peut être également préparé comme indiqué ci-après: 23,6 g (0,1 mole) de 15-méthyl-13-oxa-bicyclo[10.4.0]hexadéc-1(12)-ène et 35,0 g (0,1 mole) de tétrafluoroborate d'hydrogéno-triphényl-phosphonium dans 100 ml de xylène ont été chauffés durant 18 h à reflux. Après évaporation, on a isolé 45 g du produit désiré.

Exemple 4
Préparation du composé de formule

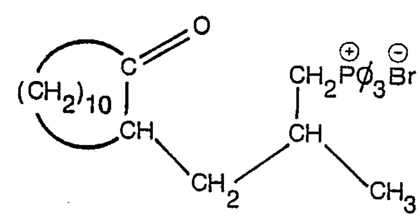

$\phi = C_6H_5$

9,8 g (0,03 mole) de 2-(2-méthyl-3-triméthylsilyloxy-prop-1-yl)-cyclododécanone et 10,3 g (0,03 mole) de bromure d'hydrogéno-triphényl-phosphonium ont été chauffés durant 15 h à une température comprise entre 140 et 150°. Après refroidissement, acidification du mélange réactionnel, traitement au moyen de $CH_2Cl_2$ et évaporation, on a isolé 16 g (rendement 95%) du produit désiré, sous forme cristalline, F. 60—90°.

IR: 1705, 1440, 1110 $cm^{-1}$
RMN: 0,9 (3H, d, J=6 Hz); 1,2 (14H, m); 1,6 (7H, m); 2,3 (2H, m); 2,7 (1H, m); 3,8 (2H, dxd, $J_1$=13 Hz, $J_2$=6 Hz); 7,7 (15H, m) $\delta$ ppm.

La 2-(2-méthyl-3-triméthyl-silyloxy-prop-1-yl)-cyclododécanone utilisée ci-dessus comme produit de départ a été obtenue selon la méthode indiquée à l'Exemple 1, à partir de cyclododécanone et 2-méthyl-3-triméthyl-silyloxy-prop-1-ène.

Eb. 120—130°/6,65 Pascals
$n_D^{20}$ : 1,4700
IR: 2899, 1709, 1466, 1361, 1247 $cm^{-1}$
RMN: 0.15 (9H, s); 0,9 (3H, d, J=6 Hz); 1,1—2,0 (20H, m); 2,2—2,9 (4H, m); 3,3 (2H, d) $\delta$ ppm.

Exemple 5
Préparation de bicyclo [10.3.0]-pentadéc-12-ène

6,6 g (0,15 mole) d'hydrure de sodium (dispersion à 55% dans l'huile minérale) ont été premièrement ajoutés goutte à goutte à 13,2 g (0,15 mole) d'alcool tert-amylique et chauffés pendant 1 h à 70°. Le mélange ainsi obtenu a ensuite été ajouté goutte à goutte et sous atmosphère d'azote à 11,35 g du sel de phosphonium obtenu à l'Exemple 1, en suspension dans 50 ml de toluène (température: 70° — addition: 30 min). Après chauffage à reflux pendant 3 h et addition de 100 ml d'eau à température ambiante, le mélange de réaction a été extrait à l'éther de pétrole (30—50). Après séchage des extraits organiques réunis, évaporation et deux distillations successives, on a isolé 2,7 g (rendement 66%) du produit désiré.

EB. 110°/6,65 Pascals
$n_D^{23}$ : 1,5078
IR: 1650, 835 $cm^{-1}$
RMN: 1,3 (20H, m), 2,2 (4H, m); 2,7 (1H, m); 5,4 (1H, m) $\delta$ ppm.

Le composé identifié ci-dessus est identique à un échantillon obtenu selon la littérature [J. Amer. Chem. Soc. *79*, 5558, (1957)].

Le bicyclo[10.3.0]pentadéc-12-ène obtenu ci-dessus peut être ensuite converti en bicyclo[10.3.0]pentadéc-1-(12)-ène comme indiqué ci-après: 191 g dudit composé dans 500 ml de toluène ont été chauffés durant 6 h à reflux, en présence de 10 g d'acide benzène-sulfonique. Après refroidissement, lavage avec $NaHCO_3$ aqueux, évaporation et distillation, on a isolé 168,2 g (rendement 88%) du produit désiré.

Eb. 80—90°/6,65 Pascals
$n_D^{23}$ : 1,5062
RMN: 1,3 (18H, m); 2,2 (8H, m) $\delta$ ppm
SM: $M^+ = 206(67)$; m/e: 135(22), 121(31), 94(52), 82(75), 81(63); 80(100), 67(57), 55(33), 41(54).

Le composé identifié ci-dessus est identique à un échantillon obtenu selon la littérature [J. Amer. Chem. Soc. *79*, 5558 (1957)].

Exemple 6

Préparation de bicyclo[10.3.0]pentadéc-12-ène

On a répété le procédé de l'Exemple 5 en faisant varier diverses conditions réactionnelles telles que nature de la base utilisée, nature du solvant et concentration. Les résultats obtenus sont rassemblés dans le tableau ci-après: dans ledit tableau, les quantités de base (g) et de solvant (ml) sont données pour 100 g de produit de départ.

| Produit de départ | Base (g) | Solvant (ml) | Conditions de réaction | Rdt (%) |
|---|---|---|---|---|
| Selon Exemple 1 1) | tert-butylate de K (21) | xylène (177) | 4 h à 120° | 73 |
| Selon Exemple 1 1) | méthylate de Na (42) | xylène/DMF 2) (195)/(59) | 4 h à 130° 3) | 74 |
| Selon Exemple 2 | tert-amylate de Na (38) | toluène (350) | 3 h à 110° | 66 |
| Selon Exemple 2 | méthylate de Na (95) | xylène/DMF 2) (877)/(88) | 3 h à 140° | 68 |
| Selon Exemple 2 1) | tert-amylate de Na (38) | xylène (175) | 20 h à 140° | 58 |

1) produit non purifié
2) diméthyl-formamide
3) distillation simultanée de méthanol

Exemple 7

Préparation de 14-méthyl-bicyclo[10.3.0]pentadéc-12-ène

34,5 g du sel de phosphonium préparé selon l'Exemple 3 ont été mis en suspension dans 100 ml de toluène et chauffés à reflux, sous atmosphère d'azote. 12,7 g (0,116 mole) de tert-amylate de sodium y ont ensuite été ajoutés, par petites portions et le chauffage à reflux prolongé pendant 4 h.

Après refroidissement, addition de 75 ml de $H_2O$, décantation, traitements successifs au moyen de $CH_2Cl_2$ et éther de pétrole (30—50) et filtration, les extraits organiques distillés ont finalement donné 6,4 g (rendement (50%) du produit désiré.

$n_D^{23}$ : 1,5042
IR: 1650, 835 $cm^{-1}$
RMN: 1,0 (15H, d, J=6 Hz); 1,1 (1,5H, d, J=6 Hz); 1,4 (18H, m); 2,1 (2H, m); 2,7 (2H, m); 5,3 (1H, m) $\delta$ ppm
SM: $M^+$ = 220(38); m/e = 205(8), 135(10), 107(45), 94(100), 93(53), 81(35), 67(20), 55(21), 41(27).

Le 14-méthyl-bicyclo[10.3.0]pentadéc-12-ène obtenu ci-dessus peut-être ensuite converti en 14-méthyl-bicyclo[10.3.0]pentadéc-1(12)-ène comme suit: 15,0 g (0,068 mole) dudit composé dans 10 ml de toluène ont été chauffés durant 6 h à reflux, en présence de 2,5 g d'acide benzènesulfonqiue. Après refroidissement, lavage avec $NaHCO_3$ aqueux, évaporation et distillation du résidu, on a recueilli 12,8 g (rendement 85%) du produit désiré, Eb. 100—110°/1,33 Pascals.

RMN: 1,0 (3H, d, J=6 Hz); 1,3 (17H, m); 2,2 (8H, m) $\delta$ ppm
SM: $M^+$ = 220(100); m/e = 205(6), 163(8); 149(20), 135(29), 121(26), 107(66), 94(98), 93(79), 81(89), 67(52), 55(67), 41(77).

Le produit identifié ci-dessus est identique à un échantillon obtenu selon la littérature [voir Chem. Abstr. *70* 88 108 v (1970)].

Dans le formules sus-mentionnées Z représente F, Cl, Br et I.

Aryl dans triaryl-phosphine est de préférence phényle ou phényle substituée par alkyle $C_1$—$C_4$ ou alkoxy $C_1$—$C_4$.

**Revendications**

1. Composés de formule

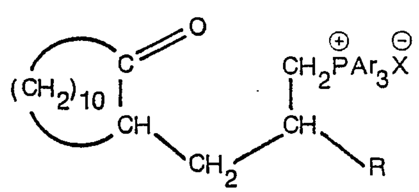

(I)

dans laquelle le symbole R représente un atome d'hydrogène ou un radical méthyle, le symbole Ar sert à désigner un radical aryle et X un atome d'halogène ou un radical $BF_4$ ou $ClO_4$.

2. Procédé pour la préparation d'un composé de formule (I), telle que définie à la revendication 1, caractérisé en ce que

A) on fait réagir un composé de formule

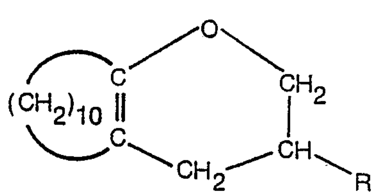

(II)

dans laquelle le symbole R est défini comme indiqué ci-dessus avec un dérivé du phosphore de formule

$$HPAr_3X \qquad (III)$$

dans laquelle le symbole Ar sert à désigner un radical aryle et X est défini comme indiqué ci-dessus; ou

B) on fait réagir un composé de formule

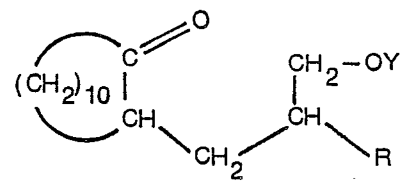

(IV)

dans laquelle le symbole R est défini comme indiqué précédemment et Y représente un radical tert-butyle, tétra-hydropyranyle ou trialkyl-silyle, avec le dérivé du phosphore de formule (III) défini ci-dessus; ou

C) on fait réagir un composé de formule

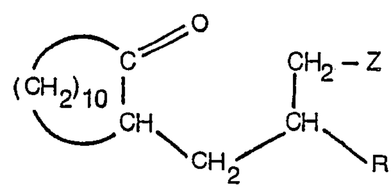

(V)

dans laquelle le symbole R est défini comme indiqué précédemment et Z représente un atome d'halogène, avec une triaryl-phosphine.

3. Utilisation d'un composé de formule (I), telle que définie à la revendication 1, à titre de produit de départ pour la préparation d'un composé de formule

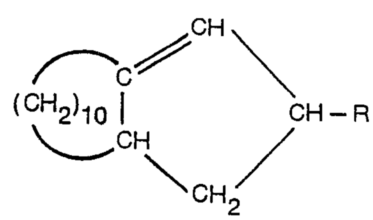

(VI)

dans laquelle le symbole R représente un atome d'hydrogène ou un radical méthyle, caractérisé en ce qu'on traite ledit composé de formule (I) au moyen d'une base.

**Claims**

1. Compounds of formula

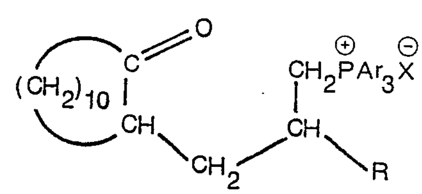

(I)

wherein symbol R represents a halogen atom or a methyl radical, symbol Ar designates an aryl radical and X stands for a halogen atom or a $BF_4$ or $ClO_4$ radical.

2. Process for the preparation of a compound of formula (I) as defined by claim 1, characterized in that

A) a compound of formula

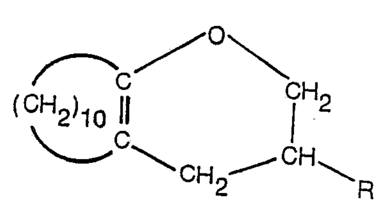

(II)

wherein symbol R is defined as indicated above, is reacted with a phosphorus derivative of formula

$$HPAr_3X \qquad (III)$$

wherein symbol Ar designates an aryl radical and X is defined as indicated above; or

B) a compound of formula

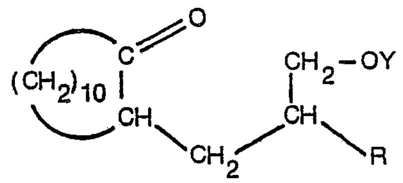

(IV)

wherein symbol R is defined as indicated above and Y represents a tert-butyl, a tetra-hydropyranyl or a trialkylsilyl radical, is reacted with the phosphorus derivative of formula (III) defined above; or

C) a compound of formula

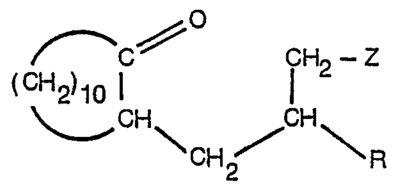

(V)

wherein symbol R is defined as indicated above and Z represents a halogen atom, is reacted with a triaryl-phosphine.

3. Utilization of a compound of formula (I), such as defined in claim 1, as starting material for the preparation of a compound of formula

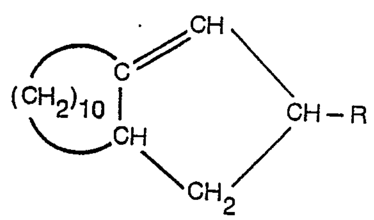

(VI)

wherein symbol R represents a hydrogen atom or a methyl radical, characterized in that the said compound of formula (I) is treated with a base.

**Patentansprüche**

1. Verbindungen der Formel

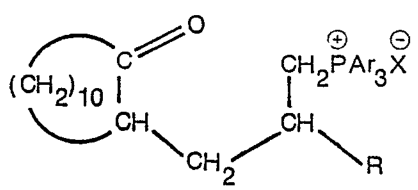

(I)

in welcher das Symbol R ein Wasserstoffatom oder ein Methylradikal bedeutet, das Symbol Ar bezeichnet ein Arylradikal und X ein Halogen, ein $BF_4$- oder ein $ClO_4$-Radikal.

2. Verfahren zur Herstellung einer Verbindung der Formel (I), wie im Anspruch 1 definiert, dadurch gekennzeichnet, dass man

A) eine Verbindung der Formel

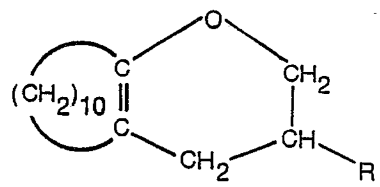

(II)

in welcher das Symbol R wie oben definiert ist, mit einem Phosphorderivat der Formel

$HPAr_3X$ (III)

in welcher das Symbol Ar ein Arylradikal bedeutet und X wie oben definiert ist, umsetzt; oder

B) eine Verbindung der Formel

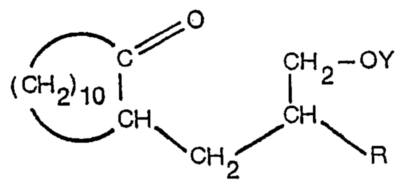

(IV)

in welcher das Symbol R wie oben definiert ist und Y ein tert-Butyl-, ein Tetrahydropyranyl- oder ein Trialkylsilylradikal darstellt, mit einer wie oben definierten Phosphorverbindung der Formel (III) umsetzt; oder

C) eine Verbindung der Formel

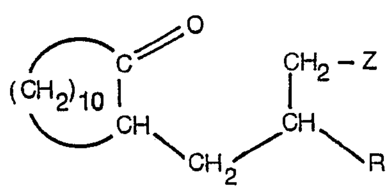

(V)

in welcher das Symbol R wie oben definiert ist und Z ein Halogenatom darstellt, mit einem Triarylphosphin umsetzt.

3. Verwendung einer Verbindung der Formel (I), wie im Patentanspruch 1 definiert, als Ausgangsprodukt zur Herstellung einer Verbindung der Formel

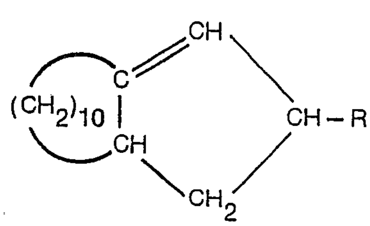

(VI)

in welcher das Symbol R ein Wasserstoffatom oder ein Methylradikal bedeutet, dadurch gekennzeichnet, dass man die genannte Verbindung der Formel (I) mit einer Base behandelt.